# EUROPEAN PATENT APPLICATION

(11) **EP 3 196 835 A1**
(43) Date of publication of application: **26.07.2017**
(21) Application number: 15838836.3
(22) Date of filing: 04.09.2015
(51) Int. Cl.: G06Q 50/22, G06Q 50/24

(54) **METHOD FOR OUTPUTTING AND INPUTTING EXAMINATION SUBJECT CLINICAL EXAMINATION DATA**

(30) Priority: 05.09.2014 JP 2014181375
(71) Applicant: Stagen Co. Ltd., Tokyo 111-0051 (JP)
(72) Inventor: KAMATANI, Naoyuki, Tokyo 167-0052 (JP); KAMATANI, Youichirou, Yokohama-shi Kanagawa 230-0012 (JP)
(74) Representative: Schwabe - Sandmair - Marx
(86) International application number: PCT/JP2015/075174
(87) International publication number: WO 2016/035877

(57) **Abstract**

One object of the present invention is to provide a technique in which an individual, without electronically connecting to the information stored in a computer of a medical institution, can safely input his clinical test data into his smartphone or wearable terminal by a method other than the manual input, and store the data as digital data. The present invention includes a system that allows conversion of the clinical test data into a barcode within the computer of the medical institution, output of the barcode on a paper, and handing over the paper to the medical examinee, and a system that allows the medical examinee to import the clinical test data by himself in the smartphone or the wearable terminal from the barcode. Moreover, the present invention includes a system that allows conversion of the clinical test data into the barcode in the computer of the medical institution, displaying the barcode on a display device, and the medical examinee to import the clinical test data by himself in the smartphone or the wearable terminal.

## Description

### [Technical Field]

The present invention relates to a method for inputting and outputting clinical test data of a medical examinee. More specifically, the present invention relates to a method for importing in a smartphone or a wearable terminal of a medical examinee the personal clinical test data of the medical examinee stored in a medical institution by using a barcode, and to a system that realizes such a method.

### [Background Art]

It is thought that in the near future many people would import and carry their own clinical test data in their own smartphone or wearable terminal and use that data for their own health maintenance. The reason is because the content of the diet can be reconsidered or the amount of the workout can be controlled after checking one's clinical test data at a given time point or by viewing a graph that shows a temporal change of the data.

In most cases, such clinical test data are generated when a person takes a medical checkup at a medical institution. The medical institution typically records such clinical test data on a paper or stores the same in a computer. The healthcare workers of course use the clinical test data to check the health of the medical examinee; however, some medical examinees also wish to use one's clinical test data.

The data at a given time point can be used to perform the health maintenance in the following manner. If the blood sugar level of the medical examinee is high, then the amount of the workout can be increased or the calorie intake can be reduced. Specifically, if the serum urate level of the medical examinee is high, the hyperuricaemia can be made normal by reducing the intake of food items rich in purine bodies. Moreover, if there is a clear increase in a cancer marker, the medical examinee can decide whether to take dedicated tests to confirm the cancer.

The data at two given time points allow monitoring of a temporal change. If the data of a medical examinee shows that the serum urate level is on increase, it is desirable to be mindful of living a lifestyle that ensures that the serum urate level does not increase further. For a medical examinee who is a cancer patient, if the cancer marker shows a gradual increase, this fact can be used to determine whether to take further treatment to cure the cancer.

The personal clinical test data is typically stored in a computer of the medical institution. A healthcare worker (mainly a doctor) performs diagnosis and treatment while referring to the clinical test data that may be printed on a paper or displayed on a display device. When explaining a medical examinee about the clinical test data, the explanation may be given merely orally or the explanation may be given while showing the medical examinee the data printed on the paper or displayed on the display device. Because the medical examinee tends to forget such explanation given to him, the clinical test data is often printed on a paper and given to the medical examinee. If the clinical test data is in data form, and if the medical examinee desires, he can manually input the data into his smartphone or wearable terminal. However, it is troublesome to manually input the data that has been printed on a paper, also, errors can arise when performing the manual input.

The method explained below of using a barcode, for example, is known in the art. When a customer purchases a product at a store and the product is to be delivered to the customer, to prevent mistakes in inputting on a delivery slip the name, address, telephone number, and the like, which are personal data of the customer, and to save labor, a method of using a barcode is known in the art (Patent Document 1). This method involves displaying on an LCD screen of a mobile telephone a barcode representing the personal data (address, name, telephone number, and the like) previously stored in the mobile telephone, and scanning the barcode displayed on the mobile telephone with a scanner of a terminal (POS) of the store thereby importing the personal data into the terminal (POS) . A delivery slip on which is printed the barcode representing the personal data is then output from a printer. Alternatively, the personal data is converted into character information by using an image recognition function, and a delivery slip on which is printed the character information is output from a printer. The prior art disclosed in the above-cited Patent Document is intended to handle only personal address and the like and such information is fundamentally different from the clinical test data. Unlike the personal information, the clinical test data is large in amount and difficult to memorize, and it changes with time and the change is important. Besides, the ethical issue of the clinical test data is very important as it relates to personal health and life.

### [Citation List]

### [Patent Document]

[Patent Document 1] Japanese Patent Application Laid-Open No. 2004-178295

### [Summary of Invention]

### [Technical Problem]

Accordingly, the most suitable method to surely input the data in a smartphone or a wearable terminal of a medical examinee is to transfer the data from a computer of a medical institution directly to the smartphone or the wearable terminal via wired or wireless communication, and the like. However, this is difficult.

The reason is because it is strictly forbidden to connect the computer of the medical institution to an external computer or a telephone equipment. Such a measure is taken based on the prior experience in which leakage of the internal data of the medical institution took place during the exchange of the data with the external device possessed the medical examinee and the like, and a computer virus was passed from the device of the medical examinee. When such an incidence happens, it is not rare that the medical treatment in the entire medical institution becomes difficult.

In view of the above discussion, it is one object of the present invention to provide a technique in which an individual, without electronically connecting to the information stored in a computer of a medical institution, can safely input his clinical test data into his smartphone or wearable terminal by a method other than the manual input, and store the data as digital data.

### [Means for Solving Problems]

The present invention includes a system that allows conversion of clinical test data into a barcode within a computer of a medical institution, output of the barcode on a paper, and handing over the paper to a medical examinee, and a system that allows the medical examinee to import the clinical test data by himself in a smartphone or a wearable terminal from the bar code.

Moreover, the present invention includes a system that allows conversion of book test data into a barcode within a computer of a medical institution, displaying the barcode on a display device, and a medical examinee to import the clinical test data by himself in a smartphone or a wearable terminal.

Concretely, the following configuration can be considered:
[1] A method of outputting clinical data of a medical examinee according to one aspect of the present invention includes following step (1) whereby the medical examinee can import his own clinical data, which has been converted into a barcode and output on a paper medium, in his smartphone or wearable terminal by himself by using an image capturing unit included in the smartphone or the wearable terminal: (1) a step of converting clinical test data of the medical examinee stored in a computer of a medical institution into the barcode.
[2] A method of outputting clinical data of a medical examinee according to another aspect of the present invention includes following steps (1) and (2) whereby the medical examinee can import his own clinical data in his smartphone or wearable terminal by himself by using an image capturing unit included in the smartphone or the wearable terminal: (1) a step of converting clinical test data of the medical examinee stored in a computer of a medical institution into a barcode; and (2) a step of outputting the barcode of the medical examinee obtained at the step (1) on a paper medium.
[3] A method of inputting and outputting clinical test data of a medical examinee according to still another aspect of the present invention includes following steps (1) to (3): (1) a step of converting the clinical test data of the medical examinee stored in a computer of a medical institution into a barcode; (2) a step of outputting the barcode of the medical examinee obtained at the step (1) on a paper medium; and (3) a step of importing in which the medical examinee imports the barcode obtained and output on the paper medium at the step (2) into his own smartphone or wearable terminal by himself by using an image capturing unit of the smartphone or the wearable terminal.
[4] A method of displaying clinical data of a medical examinee according to still another aspect of the present invention includes following step (1) whereby the medical examinee can import his own clinical data, which has been converted into a barcode and displayed on a display device, in his smartphone or wearable terminal by himself by using an image capturing unit included in the smartphone or the wearable terminal: (1) a step of converting clinical test data of the medical examinee stored in a computer of a medical institution into the barcode.
[5] A method of displaying clinical data of a medical examinee according to still another aspect of the present invention includes following steps (1) and (2) whereby the medical examinee can import his own clinical data in his smartphone or wearable terminal by himself by using an image capturing unit included in the smartphone or the wearable terminal: (1) a step of converting clinical test data of the medical examinee stored in a computer of a medical institution into a barcode; and (2) a step of displaying the barcode of the medical examinee obtained at the step (1) on a display device.
[6] A method of inputting and displaying clinical test data of a person according to still another aspect of the present invention, includes following steps (1) to (3): (1) a step of converting the clinical test data of the person stored in a computer of a medical institution into a barcode; (2) a step of displaying the barcode of the medical examinee obtained at the step (1) on a display device; and (3) a step of importing in which the medical examinee imports the barcode obtained and displayed on the display device at the step (2) into his own smartphone or wearable terminal by himself by using an image capturing unit of the smartphone or the wearable terminal.
[7] In the method according to any one of the above aspects of the present invention, the clinical test data includes at least one selected from a red blood-cell count, a white blood-cell count, a blood platelet count, total protein, a blood sugar level, HbA1c, a uric acid level, a cholesterol level, urine findings such as urine protein; and an electrocardiogram and lung function data, and does not include image data.
[8] A system for outputting clinical data of a medical examinee according to still another aspect of the present invention includes following means (1) and (2) whereby the medical examinee can import his own clinical data, which has been converted into a barcode and output on a paper medium, in his smartphone or wearable terminal by himself by using an image capturing unit included in the smartphone or the wearable terminal: (1) a computer arranged in a medical institution for storing clinical test data of the medical examinee; and (2) a system that converts the stored clinical test data of the medical examinee into the barcode.
[9] A system for outputting clinical data of a medical examinee according to still another aspect of the present invention includes following means (1) to (3) whereby the medical examinee can import his own clinical data in his smartphone or wearable terminal by himself by using an image capturing unit included in the smartphone or the wearable terminal: (1) a computer arranged in a medical institution for storing clinical test data of the medical examinee; (2) a means that converts the stored clinical test data of the medical examinee into a barcode; and (3) a means that outputs the barcode of the medical examinee on a paper medium.
[10] A system for inputting and outputting clinical test data of a medical examinee according to still another aspect of the present invention includes following means (1) to (4): (1) a computer arranged in a medical institution for storing the clinical test data of the medical examinee; (2) a means that converts the stored clinical test data of the medical examinee into a barcode; (3) a means that outputs the barcode of the medical examinee on a paper medium; and (4) at least one between a smartphone and a wearable terminal that includes an image capturing unit for importing the barcode output on the paper medium.
[11] A system for displaying clinical data of a medical examinee according to still another aspect of the present invention includes following means (1) and (2) whereby the medical examinee can import his own clinical data, which has been converted into a barcode and displayed on a display device, in his smartphone or wearable terminal by himself by using an image capturing unit included in the smartphone or wearable terminal: (1) a computer arranged in a medical institution for storing clinical test data of the medical examinee; and (2) a means that converts the stored clinical test data of the medical examinee into the barcode to be stored.
[12] A system for displaying clinical data of a medical examinee according to still another aspect of the present invention includes following means (1) to (3) whereby the medical examinee can import his own clinical data in his smartphone or wearable terminal by himself by using an image capturing unit included in the smartphone or the wearable terminal: (1) a computer arranged in a medical institution for storing clinical test data of the medical examinee; (2) a means that converts the stored clinical test data of the medical examinee into a barcode; and (3) a means that displays the barcode of the medical examinee on a display device.
[13] A system for inputting and displaying clinical test data of a person according to still another aspect of the present invention includes following means (1) to (4): (1) a computer arranged in a medical institution for storing the clinical test data of the medical examinee; (2) a means that converts the stored clinical test data of the person into a barcode; (3) a means that displays the barcode of the medical examinee on a display device; and (4) at least one between a smartphone and a wearable terminal that includes an image capturing unit for importing the barcode displayed on the display device.
[14] In the system according to any one of the above aspects of the present invention, the clinical test data includes at least one selected from a red blood-cell count, a white blood-cell count, a blood platelet count, total protein, a blood sugar level, HbA1c, a uric acid level, a cholesterol level, urine findings, an electrocardiogram, and lung function data, and does not include image data.

### [Advantageous Effects of Invention]

The present invention provides the following advantageous effects.
(1) because data is not input manually, an error does not occur
(2) the personal information is protected
   Even today the doctors are handing over the printed personal data to a medical examinee on a daily basis, so that the act of converting the data into a barcode and handing it over to a medical examinee does not pose any additional threat to protection of private information.
(3) Because the computer of the medical institution and the device of the medical examinee are not connected electronically, there is no issue of computer failure.

The present invention is fundamentally different from the technologies in which the barcode is used for data other than the clinical test data. Firstly, unlike the personal information such as address and telephone number, the clinical test data is large in amount and difficult to memorize. Moreover, the clinical test data changes with time and the change is important. Besides, the ethical issue of the clinical test data is very important as it relates to personal health and life.

### [Brief Description of Drawings]

FIG. 1 shows a schematic diagram of a situation in which clinical test data of a medical examinee previously stored in a computer of a medical institution is converted into a barcode, displayed on a display device, printed on a paper, and the paper is handed-over to the medical examinee.
FIG. 2 shows a schematic diagram of an importing step in which the medical examinee imports the barcode printed on the paper in his smartphone. Though the smartphone has been shown in this diagram, a wearable terminal may be used instead of the smartphone.
FIG. 3 shows a schematic diagram of an importing step in which the medical examinee imports the barcode displayed on the display device in his smartphone. Though the smartphone has been shown in this diagram, a wearable terminal may be used instead of the smartphone.
FIG. 4 shows a schematic diagram of a screen of the smartphone that depicts that data converted into a barcode is imported in the smartphone. Though the smartphone has been shown in this diagram, a wearable terminal may be used instead of the smartphone.
FIG. 5 shows a schematic diagram of a screen of the smartphone that depicts that data corresponding to the barcode that was imported in the smartphone has been restored into the digital data obtained in the actual clinical test. Though the smartphone has been shown in this diagram, a wearable terminal may be used instead of the smartphone.
FIG. 6 shows a schematic diagram of a screen of the smartphone that depicts that the imported clinical test data is registered in the smartphone. Though the smartphone has been shown in this diagram, a wearable terminal may be used instead of the smartphone.
FIG. 7 shows a schematic diagram of a screen of the smartphone that depicts that the imported clinical test data is registered in the smartphone. Though the smartphone has been shown in this diagram, a wearable terminal may be used instead of the smartphone.
FIG. 8 shows a schematic diagram of a screen of the smartphone that depicts a graph of a temporal change of the clinical test data generated by a smartphone application from clinical test data stored previously in the smartphone and the clinical test data imported this time of the medical examinee. Though the smartphone has been shown in this diagram, a wearable terminal may be used instead of the smartphone.
FIG. 9 depicts a system configuration of the medical institution shown in FIG. 1.
FIG. 10 is a structural diagram of a configuration of the smartphone used to read a barcode.
FIG. 11 is a flowchart indicating a process procedure for reading clinical test data using a barcode.
FIG. 12 is an explanatory drawing for explaining how clinical test data is used in a smartphone.

### [Description of Embodiments]

The clinical test data is important to an individual. The personal clinical test data is often stored in a computer of a medical institution. Currently, the clinical test data is printed on a paper and the paper is given to a medical examinee. It will be convenient if the medical examinee can input his data into his smartphone or wearable terminal. However, it is troublesome to manually input the data that has been printed on the paper, also, errors can arise during the input. The most suitable method to avoid such errors is to directly transfer the data from the computer of the medical institution to the smartphone or the wearable terminal of the medical examinee by using wired or wireless communication; however, this is difficult. The reason is because it is strictly forbidden to connect the computer of the medical institution to an external computer or a telephone equipment. Such a measure is taken based on the prior experience in which leakage of the internal data took place and a computer virus was passed during the exchange of the data with the external device possessed by the medical examinee and the like.

The present invention relates to a system that converts clinical test data stored in the computer of the medical institution into a barcode, and prints the clinical test data converted into the barcode on a paper. The paper is handed-over to the medical examinee. The medical examinee can himself import the clinical test data converted into the barcode into his smartphone or wearable terminal and use it to perform his own health management.

Instead of printing the barcode on a paper, the medical institution can display the barcode on a display device, and the medical examinee can capture an image of the displayed barcode with his smartphone or wearable terminal thereby importing the data.

### Smartphone and Wearable Terminal

The smartphone used in the present invention is a multifunctional mobile telephone.

The wearable terminal used in the present invention is a computer that can be carried by wearing. The wearable terminal is also called a wearable device or a wearable computer.

### Method of Generating Barcode

An existing system can be used as a system for converting character or numerical clinical test data into a barcode and restoring the barcode to the original data, or such a system can be developed newly. A medical institution can convert the clinical test data of a person into a barcode by using a simple computer program.

Unless specified differently, the barcode mentioned in the present specification refers to a two-dimensional barcode. Moreover, the barcode mentioned in the present specification includes a QR code (registered trademark) in addition to the conventional barcode made of lines.

### Method for Importing Information Converted into Barcode in Smartphone or Wearable Terminal

An existing method can be used as a method for importing a barcode, which has been printed on a paper or which has been displayed on a screen of a computer of a medical institution, in a smartphone or a wearable terminal, or such a method can be developed newly. Specifically, a method of capturing an image of a barcode with an image capturing unit of a smartphone or a wearable terminal and restoring digital data from the captured image can be mentioned as an example of such a method.

### Clinical Test Data

The clinical test data includes hematologic data such as a red blood-cell count, a white blood-cell count, a blood platelet count; blood biochemistry data such as total protein, a blood sugar level, HbA1c, a uric acid level, a cholesterol level; urine findings such as urine protein; and physiologic test data such as an electrocardiogram and lung function data. The clinical test data does not include image data and the like.

### Embodiments

A method for realizing the present invention is explained below.

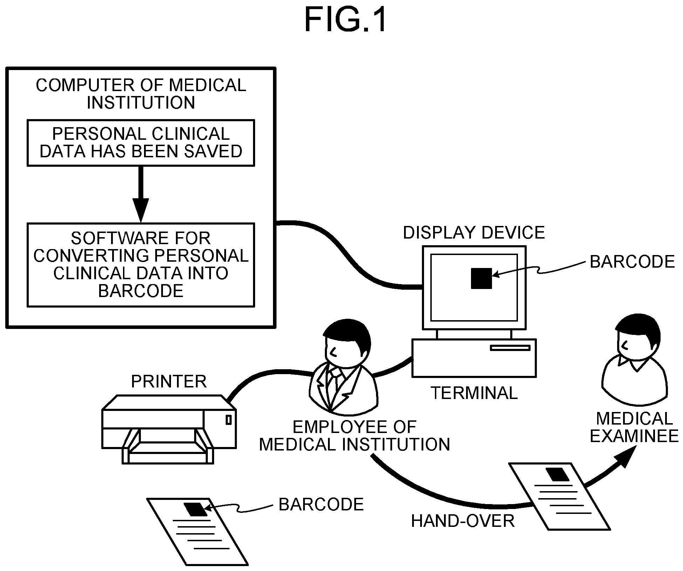

FIG. 1 shows a process procedure in which clinical test data and the like of a medical examinee previously stored in a computer of a medical institution is converted into a barcode, and the information converted into the barcode is handed-over to the medical examinee.
(1) The medical institution has the computer for saving the clinical test data of the medical examinee, and a software for converting the stored clinical test data into a barcode. An employee (in many cases a doctor) of the medical institution prints the clinical test data converted into the barcode on a paper. At this time, the raw clinical test data (the data before converting into the barcode) can also be printed on the paper, if desired.
(2) As shown in FIG. 1, the employee of the medical institution hands-over the barcode printed paper to the medical examinee.
(3) As shown in FIG. 1, when the employee of the medical institution displays the barcode on a display device, the medical examinee can capture an image of the barcode by using his smartphone. Though the smartphone has been shown in this diagram, a wearable terminal may be used instead of the smartphone.

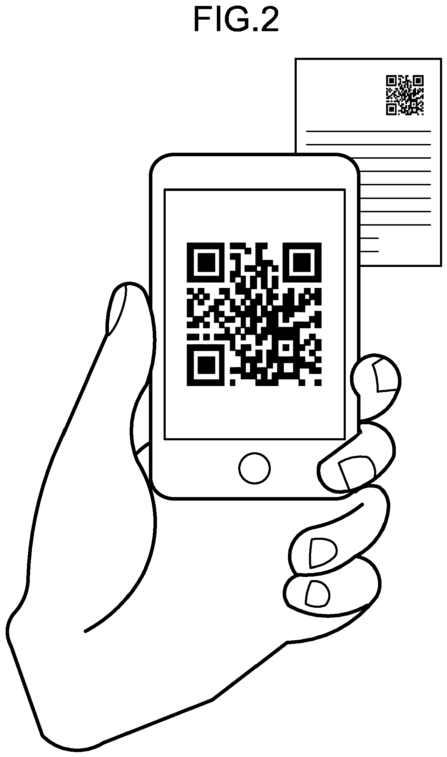

FIG. 2 shows a situation in which the medical examinee imports the barcode printed on the paper in his smartphone. The medical examinee can capture the image of the barcode by using an image capturing unit included in his smartphone thereby importing the barcode. Moreover, if a scanner function is available, the barcode can be imported from the paper by using the scanner function. Though the smartphone has been shown in this diagram, a wearable terminal may be used instead of the smartphone.

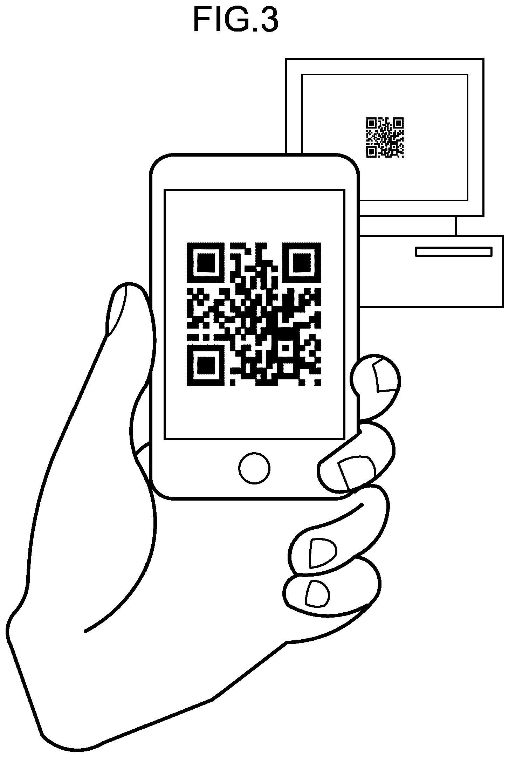

FIG. 3 shows a situation in which the medical examinee imports the barcode displayed on a display device in his smartphone. The medical examinee can capture the image of the barcode by using an image capturing unit included in his smartphone thereby importing the barcode. Though the smartphone has been shown in this diagram, a wearable terminal may be used instead of the smartphone.

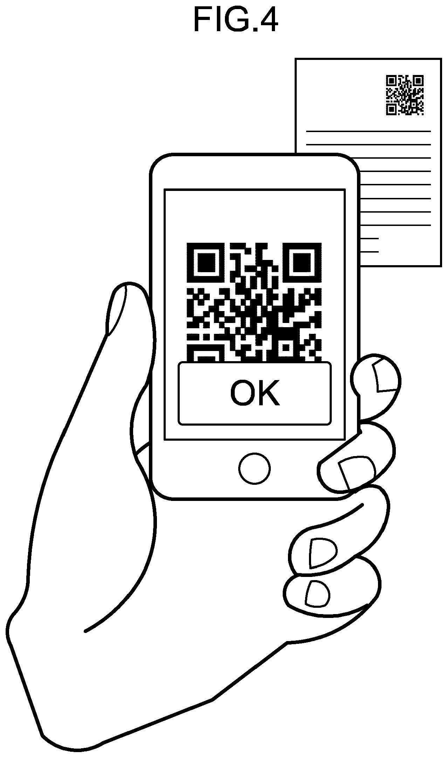

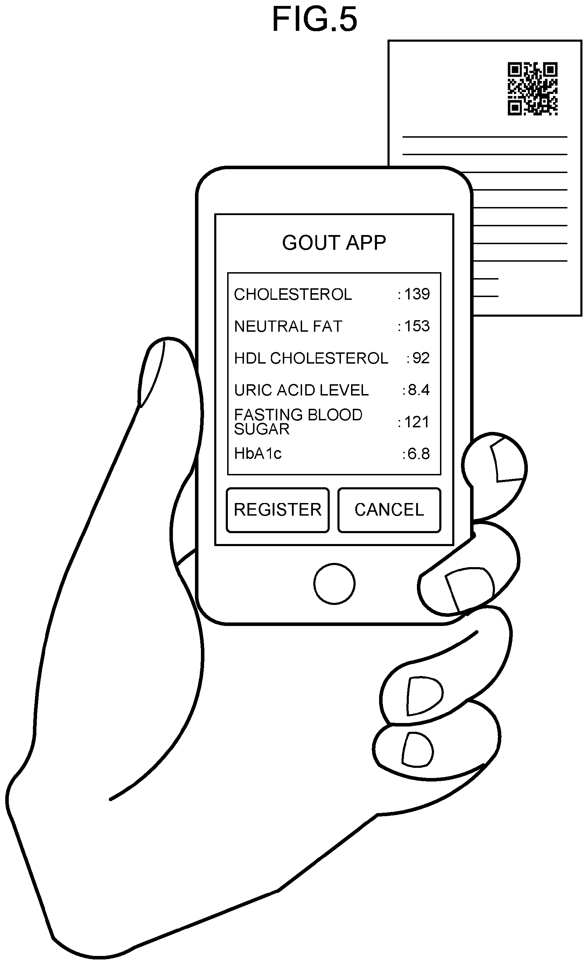

FIG. 4 shows a screen of the smartphone that depicts that data of the barcode has been imported in the smartphone. FIG. 5 shows a screen of the smartphone that depicts that the data of the barcode that was imported in the smartphone has been restored into the actual clinical test data (digital data). Though the smartphone has been shown in this diagram, a wearable terminal may be used instead of the smartphone.

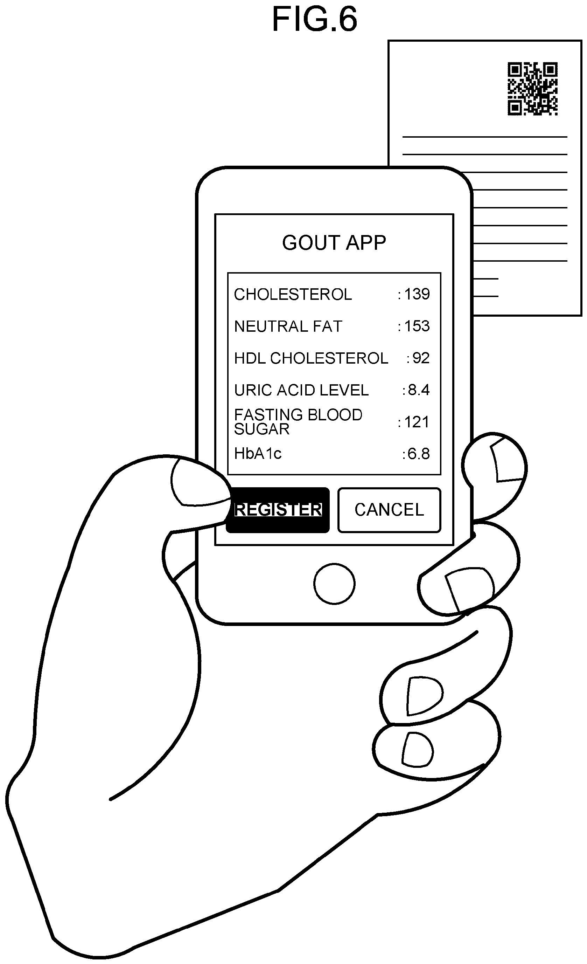

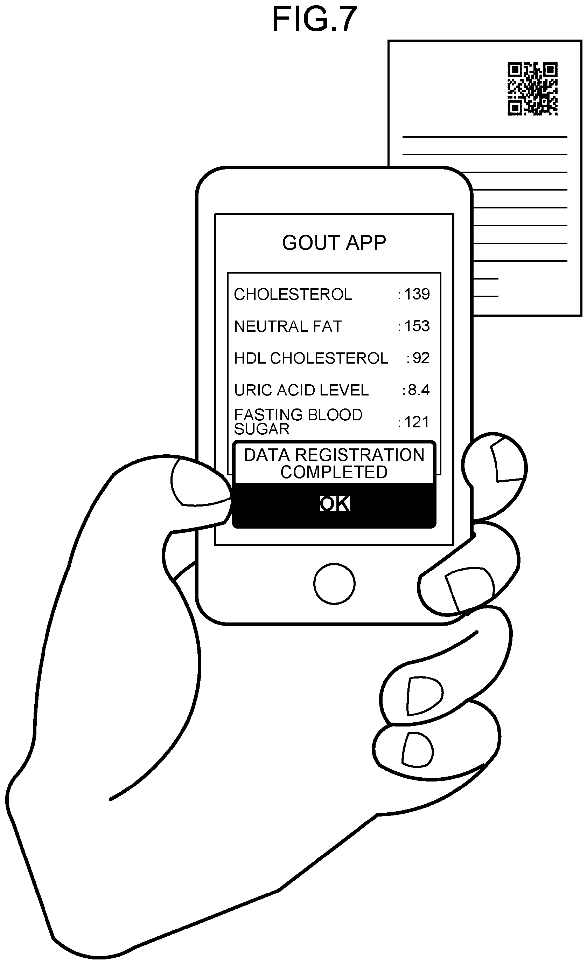

FIGS. 6 and 7 show screens of the smartphone that depict that the clinical test data restored as the digital data is registered as data in the smartphone of the medical examinee. Though the smartphone has been shown in this diagram, a wearable terminal may be used instead of the smartphone.

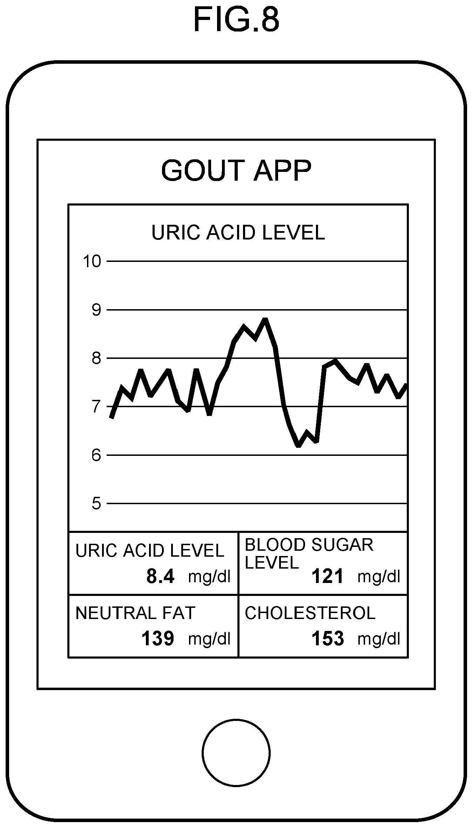

FIG. 8 shows a screen of the smartphone that depicts a graph of a temporal change of the clinical test data of the medical examinee generated by a smartphone application from clinical test data stored previously in the smartphone and the clinical test data imported this time. Though the smartphone has been shown in this diagram, a wearable terminal may be used instead of the smartphone.

In this manner, the present invention presents a method including a step of converting the clinical test data stored in the computer of the medical institution into the barcode. Moreover, the method includes a step of printing the barcode converted data on a paper and handing over the paper to the medical examinee. Besides, the method includes a step of importing in which the medical examinee imports in his smartphone or wearable terminal the barcode printed on the paper by using the image capturing unit included in the smartphone or the wearable terminal.

Instead of printing the barcode on a paper, the medical institution can display the barcode on a display device, and the medical examinee can capture an image of the displayed barcode with his smartphone or wearable terminal thereby importing the data.

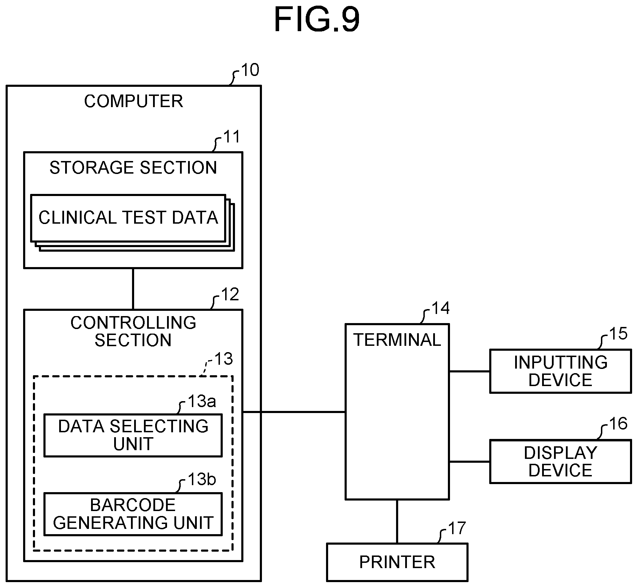

A system configuration of the medical institution shown in FIG. 1 is explained below. FIG. 9 depicts the system configuration of the medical institution shown in FIG. 1. As shown in FIG. 9, a terminal 14 is connected to a computer 10, and an inputting device 15, a display device 16, and a printer 17 are connected to the terminal 14.

The computer 10 is installed in the medical institution, for example, and it is used to perform integrated management of various data in the medical institution. A plurality of the terminals 14 is arranged in the medical institution and a plurality of employees of the medical institution can operate these terminals 14 simultaneously, for example.

The computer 10 includes a storage section 11 and a controlling section 12. The storage section 11 is a storage device such as a hard disk drive or the nonvolatile memory, and it is used to store therein a plurality of pieces of clinical test data. The clinical test data is mapped with identification information and the like of a medical examinee.

The controlling section 12 is an arithmetic processing unit such as a CPU (Central Processing Unit). The controlling section 12 can realize a function by executing a predetermined application program corresponding to the function.

In the example shown in FIG. 9, the controlling section 12 includes an application 13 for converting the clinical test data into a barcode. A function of each of a data selecting unit 13a and a barcode generating unit 13b is realized when the application 13 is executed by the controlling section 12.

The data selecting unit 13a is a processing unit that selects processing target data from the clinical test data. For example, when the employee operates the terminal 14 and specifies a particular medical examinee, the data selecting unit 13a selects the clinical test data mapped with the specified medical examinee. At this time, it is allowable to select all the pieces of the clinical test data mapped with that medical examinee or to extract only those pieces of the clinical test data that satisfy a predetermined condition. For example, conditions such as a fact that it is possible to generate a barcode, a fact that a certain test result is out of its normal range, a fact that a test item is associated with the particular disease can be used as the condition.

The barcode generating unit 13b is a processing unit that encodes the clinical test data selected by the data selecting unit 13a to generate a barcode. The barcode generating unit 13b transmits the barcode generated by the encoding to the requester terminal 14.

The terminal 14 is operated by the employee of the medical institution and gives the employee access to various data managed by the computer 10. The inputting device 15 is an input interface, such as a keyboard and a mouse, and it is used by the employee when performing operation or inputting data. The display device 16 is an output interface used for displaying various data. The printer 17 is an output interface used for printing various data.

The terminal 14 can specify a medical examinee, for example, and acquire the clinical test data of the specified medical examinee from the computer 10. The computer 10 can convert a part of or all of the clinical test data into the barcode before the data is acquired by the terminal 14.

The terminal 14 causes the display device 16 to display the acquired clinical test data or the barcode. The terminal 14 can cause the printer 17 to print the acquired clinical test data or the barcode. The medical examinee can read the displayed or the printed barcode by using the smartphone and the like.

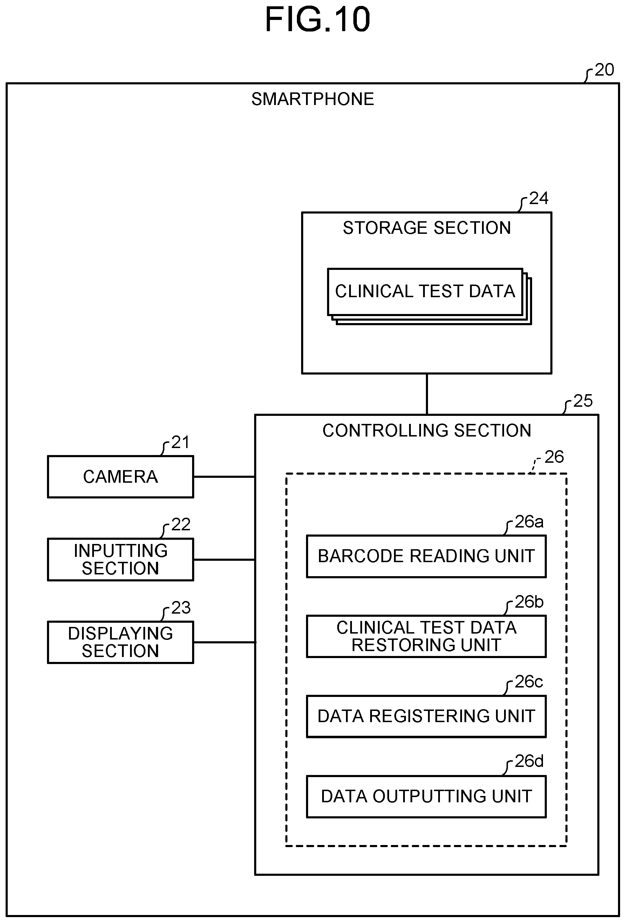

FIG. 10 is a structural diagram of a configuration of the smartphone used to read the barcode. As shown in FIG. 10, a smartphone 20 includes a camera 21, an inputting section 22, a displaying section 23, a storage section 24, and a controlling section 25.

The camera 21 is a capturing device used for capturing an image of a barcode. The inputting section 22 is an input interface, such as a key or a touch panel, used for performing an input operation by the medical examinee. The displaying section 23 is an output interface such as a liquid crystal panel. The inputting section 22 and the displaying section 23 can be integrated to form a touch panel display. In addition, the smartphone 20 includes a voice outputting interface, a voice inputting interface, a communication interface, and the like.

The storage section 24 is a storage device, such as a hard disk drive or a nonvolatile memory, and is used to store a plurality of pieces of the clinical test data. This clinical test data is acquired from a barcode and registered. Moreover, it is preferable that the clinical test data is mapped with the date and the like of the test.

The controlling section 25 is an arithmetic processing unit, such as a CPU, and can realize a function by executing a predetermined application program corresponding to the function.

In the example shown in FIG. 10, the controlling section 25 includes an application 26 for processing the clinical test data. A function of each of a barcode reading unit 26a, a clinical test data restoring unit 26b, a data registering unit 26c, and a data outputting unit 26d is realized when the application 26 is executed by the controlling section 25.

The barcode reading unit 26a is a processing unit that recognizes a barcode in the image captured by using the camera 21, and reads the barcode. The clinical test data restoring unit 26b is a processing unit that restores the clinical test data by decoding the read barcode.

The data registering unit 26c performs a process of registering the clinical test data, which is restored by the clinical test data restoring unit 26b, in the storage section 24. At this time, if the date of the test is included in the clinical test data itself, the data registering unit 26c registers the date by mapping with the clinical test data. If the date of the test is not included in the clinical test data itself, the data registering unit 26c registers the date inputted by the medical examinee or the date on which the clinical test data was read by mapping with the clinical test data.

The data outputting unit 26d is a processing unit for outputting the clinical test data. The data outputting unit 26d can perform a display control to display the clinical test data, which is restored by the clinical test data restoring unit 26b, as is on the displaying section 23. Moreover, the data outputting unit 26d generates a graph indicating a change in the test values from a plurality of pieces of the clinical test data and dates registered in the storage section 24, and performs a display control to display a graph showing change of the test values on the displaying section 23.

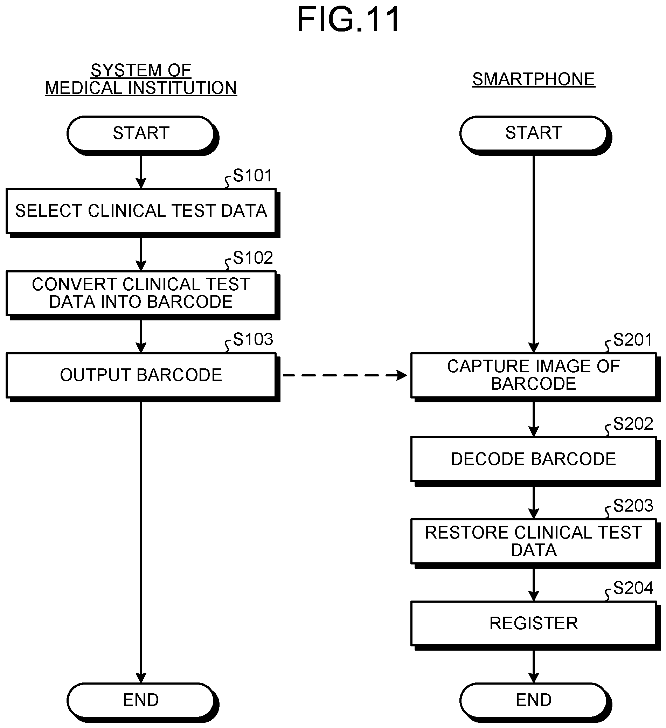

A process procedure for reading clinical test data from a barcode is explained below. FIG. 11 is a flowchart indicating the process procedure for reading the clinical test data from the barcode. At first, the data selecting unit 13a of the computer 10 selects clinical test data of a medical examinee specified from the terminal 14 (Step S101).

The barcode generating unit 13b of the computer 10 converts the clinical test data selected by the data selecting unit 13a into a barcode (Step S102). Thus obtained barcode is output by displaying on the display device 16 or by printing by using the printer 17 (Step S103).

The camera 21 of the smartphone 20 captures an image of the output barcode (Step S201). The image-captured barcode is read by the barcode reading unit 26a. The clinical test data restoring unit 26b restores the clinical test data (step S203) by decoding the barcode (step S202). The data registering unit 26c registers the restored clinical test data in the storage section 24 (Step S204), and the process procedure is terminated.

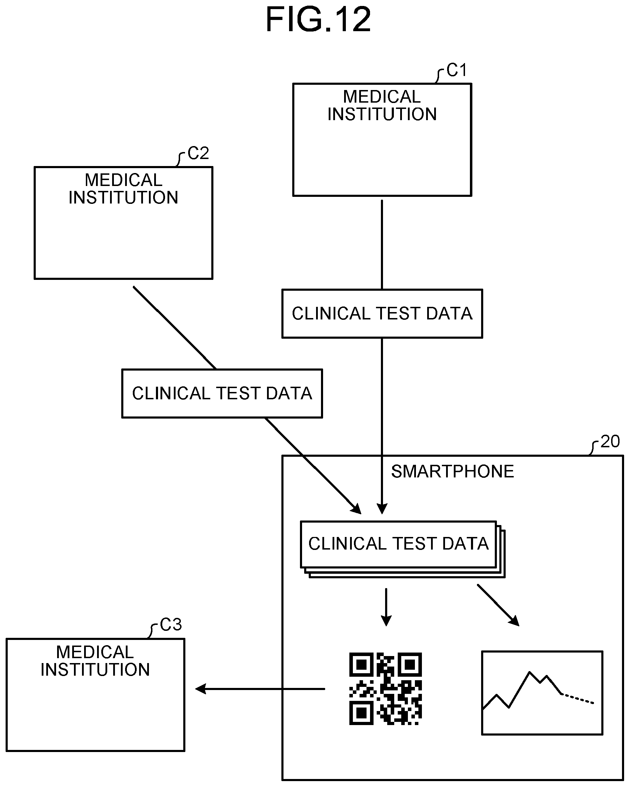

How the clinical test data is used in the smartphone 20 is explained below. FIG. 12 is an explanatory drawing for explaining how the clinical test data is used in the smartphone 20. The smartphone 20 can acquire and accumulate the clinical test data from a plurality of medical institutions.

FIG. 12 shows a situation in which a medical check-up is performed in each of a medical institution C1 and a medical institution C2, and the acquired clinical test data are accumulated in the smartphone 20. By doing so, even if the medical check-up has been performed in different medical institutions, by accumulating the test results for the same test item from the medical institutions, it is possible to generate a graph that shows a temporal change of the test item.

Moreover, how the test value is expected to change in future can be predicted from a plurality of pieces of the clinical test data, and the forecast can be displayed. When displaying the forecast of the change, it is preferable that it is displayed in a different manner. In the example shown in FIG. 12, the temporal change of the test values in the clinical test data is shown with a continuous line while the forecast of the test value change is shown with a dashed line.

If the clinical test data are acquired from different medical institutions, it is allowable to display the data from each of the medical institutions in different manner.

The clinical test data accumulated in the smartphone 20 can be offered to a medical institution. As shown in FIG. 12, if a barcode generated from the clinical test data is displayed on the smartphone 20, an image of the barcode can be captured in a medical institution C3 thereby reading the clinical test data.

As explained above, in the method according to the present embodiment, because the exchange of the clinical test data is performed by using a barcode, personal information can be protected, high level of security can be maintained, and the data can be exchanged correctly.

The present invention should not be interpreted so as to be limited to the above explained embodiments, and the embodiment can be changed appropriately. For example, when determining the clinical test data to be converted into a barcode, the medical examinee can be allowed to select a different test item. Specifically, a configuration is allowable in which a test item set previously by the medical examinee is always included as a target for converting into a barcode. Moreover, a configuration is allowable in which a test item that is related to a test item set previously by the medical examinee is selected automatically. Moreover, a configuration is allowable in which if there exists, in the plural pieces of the clinical test data that have been already registered in the smartphone and the like, a test item that is out of its appropriate range, that test item is selected automatically.

Moreover, if it is possible to perform data communication among a plurality of medical institutions via a safe telecommunication line, then those medical institutions can cooperate with each other, a given medical institution can be allowed to acquire the clinical test data stored in other medical institutions, convert the data into a barcode and output the barcode. In this configuration, it is preferable that the data to be converted into a barcode is acquired selectively, and the clinical test data acquired from the other medical institutions is deleted after the data is presented to the medical examinee. Moreover, it may be decided to acquire the clinical test data from other medical institutions with the proviso that such a secured environment is realized.

### [Industrial Applicability]

According to the present invention, an individual, without electronically connecting to the information stored in a computer of a medical institution, can safely input his clinical test data into his smartphone or wearable terminal by a method other than the manual input, and store the inputted data as digital data. This digital data can be displayed together with data measured over time, a graph can be plotted by using the data, and the data can be used for own health management.

### [Explanation of Reference Numerals]

- 10: Computer
- 11, 24: Storage section
- 12, 25: Controlling section
- 13, 26: Application
- 13a: Data selecting unit
- 13b: Barcode generating unit
- 14: Terminal
- 15: Inputting device
- 16: Display device
- 17: Printer
- 20: Smartphone
- 21: Camera
- 22: Inputting section
- 23: Displaying section
- 26a: Barcode reading unit
- 26b: Clinical test data restoring unit
- 26c: Data registering unit
- 26d: Data outputting unit

## Claims

1. A method of outputting clinical data of a medical examinee comprising following step (1) whereby the medical examinee can import his own clinical data, which has been converted into a barcode and output on a paper medium, in his smartphone or wearable terminal by himself by using an image capturing unit included in the smartphone or the wearable terminal:
(1) a step of converting clinical test data of the medical examinee stored in a computer of a medical institution into the barcode.

2. A method of outputting clinical data of a medical examinee comprising following steps (1) and (2) whereby the medical examinee can import his own clinical data in his smartphone or wearable terminal by himself by using an image capturing unit included in the smartphone or the wearable terminal:
(1) a step of converting clinical test data of the medical examinee stored in a computer of a medical institution into a barcode; and
(2) a step of outputting the barcode of the medical examinee obtained at the step (1) on a paper medium.

3. A method of inputting and outputting clinical test data of a medical examinee comprising following steps (1) to (3):
(1) a step of converting the clinical test data of the medical examinee stored in a computer of a medical institution into a barcode;
(2) a step of outputting the barcode of the medical examinee obtained at the step (1) on a paper medium; and
(3) a step of importing in which the medical examinee imports the barcode obtained and output on the paper medium at the step (2) into his own smartphone or wearable terminal by himself by using an image capturing unit of the smartphone or the wearable terminal.

4. A method of displaying clinical data of a medical examinee comprising following step (1) whereby the medical examinee can import his own clinical data, which has been converted into a barcode and displayed on a display device, in his smartphone or wearable terminal by himself by using an image capturing unit included in the smartphone or the wearable terminal:
(1) a step of converting clinical test data of the medical examinee stored in a computer of a medical institution into the barcode.

5. A method of displaying clinical data of a medical examinee comprising following steps (1) and (2) whereby the medical examinee can import his own clinical data in his smartphone or wearable terminal by himself by using an image capturing unit included in the smartphone or the wearable terminal:
(1) a step of converting clinical test data of the medical examinee stored in a computer of a medical institution into a barcode; and
(2) a step of displaying the barcode of the medical examinee obtained at the step (1) on a display device.

6. A method of inputting and displaying clinical test data of a person, comprising following steps (1) to (3):
(1) a step of converting the clinical test data of the person stored in a computer of a medical institution into a barcode;
(2) a step of displaying the barcode of the medical examinee obtained at the step (1) on a display device; and
(3) a step of importing in which the medical examinee imports the barcode obtained and displayed on the display device at the step (2) into his own smartphone or wearable terminal by himself by using an image capturing unit of the smartphone or the wearable terminal.

7. The method according to any one of claims 1 to 6, wherein the clinical test data includes at least one selected from a red blood-cell count, a white blood-cell count, a blood platelet count, total protein, a blood sugar level, HbA1c, a uric acid level, a cholesterol level, urine findings such as urine protein; and an electrocardiogram and lung function data, and does not include image data.

8. A system for outputting clinical data of a medical examinee comprising following means (1) and (2) whereby the medical examinee can import his own clinical data, which has been converted into a barcode and output on a paper medium, in his smartphone or wearable terminal by himself by using an image capturing unit included in the smartphone or the wearable terminal:
(1) a computer arranged in a medical institution for storing clinical test data of the medical examinee; and
(2) a system that converts the stored clinical test data of the medical examinee into the barcode.

9. A system for outputting clinical data of a medical examinee comprising following means (1) to (3) whereby the medical examinee can import his own clinical data in his smartphone or wearable terminal by himself by using an image capturing unit included in the smartphone or the wearable terminal:
(1) a computer arranged in a medical institution for storing clinical test data of the medical examinee;
(2) a means that converts the stored clinical test data of the medical examinee into a barcode; and
(3) a means that outputs the barcode of the medical examinee on a paper medium.

10. A system for inputting and outputting clinical test data of a medical examinee comprising following means (1) to (4):
(1) a computer arranged in a medical institution for storing the clinical test data of the medical examinee;
(2) a means that converts the stored clinical test data of the medical examinee into a barcode;
(3) a means that outputs the barcode of the medical examinee on a paper medium; and
(4) at least one between a smartphone and a wearable terminal that includes an image capturing unit for importing the barcode output on the paper medium.

11. A system for displaying clinical data of a medical examinee comprising following means (1) and (2) whereby the medical examinee can import his own clinical data, which has been converted into a barcode and displayed on a display device, in his smartphone or wearable terminal by himself by using an image capturing unit included in the smartphone or wearable terminal:
(1) a computer arranged in a medical institution for storing clinical test data of the medical examinee; and
(2) a means that converts the stored clinical test data of the medical examinee into the barcode to be stored.

12. A system for displaying clinical data of a medical examinee comprising following means (1) to (3) whereby the medical examinee can import his own clinical data in his smartphone or wearable terminal by himself by using an image capturing unit included in the smartphone or the wearable terminal:
(1) a computer arranged in a medical institution for storing clinical test data of the medical examinee;
(2) a means that converts the stored clinical test data of the medical examinee into a barcode; and
(3) a means that displays the barcode of the medical examinee on a display device.

13. A system for inputting and displaying clinical test data of a person comprising following means (1) to (4):
(1) a computer arranged in a medical institution for storing the clinical test data of the medical examinee;
(2) a means that converts the stored clinical test data of the person into a barcode;
(3) a means that displays the barcode of the medical examinee on a display device; and
(4) at least one between a smartphone and a wearable terminal that includes an image capturing unit for importing the barcode displayed on the display device.

14. The system according to any one of claims 8 to 13, wherein the clinical test data includes at least one selected from a red blood-cell count, a white blood-cell count, a blood platelet count, total protein, a blood sugar level, HbA1c, a uric acid level, a cholesterol level, urine findings, an electrocardiogram, and lung function data, and does not include image data.
